# EUROPEAN PATENT APPLICATION

(11) **EP 4 233 992 A1**
(43) Date of publication of application: **30.08.2023**
(21) Application number: 21897892.2
(22) Date of filing: 22.11.2021
(51) Int. Cl.: A61N 1/08, A61B 18/12

(54) **HIGH-FREQUENCY TREATMENT DEVICE AND HIGH-FREQUENCY TREATMENT METHOD**

(30) Priority: 30.11.2020 JP 2020198192
(71) Applicant: Kabushiki Kaisha Top, Adachi-ku, Tokyo 120-0035 (JP)
(72) Inventor: IKEUCHI, Atsushi, Tokyo 120-0035 (JP)
(74) Representative: Schön, Christoph
(86) International application number: PCT/JP2021/042709
(87) International publication number: WO 2022/113919

(57) **Abstract**

There is provided a high-frequency treatment device and a high-frequency treatment method capable of appropriately performing a treatment by measuring a treatment temperature with high accuracy. A high-frequency treatment device 1 includes output units 41 and 42 that output high-frequency power, a plurality of electrodes 21 to 24 that is connected to the output units 41, 42 and disposed at a treatment target, temperature sensors 21c to 24c provided at the electrodes 21 to 24 or disposed in the vicinity of the electrodes 21 to 24, temperature measurement units 61 and 62 that generate a temperature measurement signal based on a signal received from the temperature sensors 21c to 24c, a temperature correction unit 91 that corrects the temperature measurement signal based on correction information on an individual characteristic of a component related to the temperature measurement to generate a correction temperature signal or calculate a temperature measurement value, and a control unit 80 that controls the output of the output units 41, 42 based on the correction temperature signal or the temperature measurement value.

## Description

### Technical Field

The present invention relates to a high-frequency treatment device and a high-frequency treatment method for performing a treatment such as thermal coagulation of nerve tissue or ablation of tumor tissue by applying a high-frequency current to a treatment target such as a human or an animal.

### Background Art

In the related art, in the medical field, a high-frequency treatment in which an electrode is disposed in a body of a human or an animal and a high-frequency current is passed from the electrode to perform ablation of a tissue or the like is widely used. Further, in recent years, as one of pain treatments, a method of performing nerve blocking by a high-frequency treatment has attracted attention (for example, refer to Patent Literature 1). The nerve blocking by the high-frequency treatment has advantages in that there are few side effects on surrounding tissues and the effect lasts for a long period, as compared with the method using a drug in the related art.

There are two types of nerve blocking by this high-frequency treatment, these methods are a high-frequency thermal coagulation method and a pulse high-frequency method, and the high-frequency thermal coagulation method is a method of heating a part of a nerve tissue at a temperature of approximately 80°C for several minutes with a high-frequency current flowing from electrodes and thermally coagulating the nerve tissue, thereby blocking pain signals. In addition, the pulse high-frequency method is a method in which a high-frequency current is intermittently passed through a part of a nerve tissue to heat the nerve tissue at a temperature of 42°C or lower for a dozen minutes, thereby blocking pain signals without damaging the nerve.

Both the high-frequency thermal coagulation method and the pulse high-frequency method are performed by heating a part of the nerve tissue at a predetermined treatment temperature for a predetermined treatment time. Therefore, in the high-frequency treatment, an ambient temperature of the electrode is measured by a temperature sensor incorporated in the electrode, and an output of high-frequency power is controlled so that a temperature measurement value is equal to a preset temperature setting value (control target value).

### Citation List

### Patent Literature

Patent Literature 1: PCT Japanese Translation Patent Publication No. 2018-511444

### Summary of Invention

### Technical Problem

Meanwhile, in general, various components that transmit, amplify, or the like a measurement signal, including a temperature sensor such as a thermocouple or a temperature measurement resistor, have individual differences and different characteristics. Therefore, there is a possibility that an error may occur in the temperature measurement value even in the high-frequency treatment.

Meanwhile, in a high-frequency treatment device in the related art, since the error caused by the individual characteristics of such a component is not taken into consideration, heating to the nerve tissue may become insufficient or excessive even in a case where output control is appropriately performed. In other words, there is a problem in which although a treatment is not performed at an appropriate temperature, this cannot be checked during the treatment.

In view of such circumstances, the present invention has an object to provide a high-frequency treatment device and a high-frequency treatment method capable of appropriately performing a treatment by measuring a treatment temperature with high accuracy.

### Solution to Problem

According to an aspect of the present invention, there is provided a high-frequency treatment device including: an output unit configured to output high-frequency power; a plurality of electrodes connected to the output unit and disposed at a treatment target; a temperature sensor provided at the electrode or disposed in a vicinity of the electrode; a temperature measurement unit configured to generate a temperature measurement signal based on a signal received from the temperature sensor; a temperature correction unit configured to correct the temperature measurement signal based on correction information on an individual characteristic of a component related to the temperature measurement to generate a correction temperature signal or calculate a temperature measurement value; and a control unit configured to control the output of the output unit based on the correction temperature signal or the temperature measurement value.

In addition, according to another aspect of the present invention, there is provided a high-frequency treatment method of outputting high-frequency power to a plurality of electrodes disposed at a treatment target to perform a treatment, the method including: a temperature measurement process of generating a temperature measurement signal based on a signal received from a temperature sensor provided at the electrode or disposed in a vicinity of the electrode; a temperature correction process of correcting the temperature measurement signal based on correction information on an individual characteristic of a component related to the temperature measurement to generate a correction temperature signal or calculate a temperature measurement value; and an output control process of controlling the output of the high-frequency power based on the correction temperature signal or the temperature measurement value.

With the present invention, by correcting the temperature measurement signal with the correction information, it is possible to cancel an error caused by the individual difference of the component related to the temperature measurement. Accordingly, it is possible to measure the ambient temperature of the electrode, that is, a treatment temperature with high accuracy and control the output of the high-frequency power. Therefore, it is possible to accurately maintain a preset treatment temperature for heating a nerve tissue and appropriately perform the treatment.

Further, the high-frequency treatment device according to the aspect of the present invention, further includes: a main body configured to include the output unit, the temperature measurement unit, the temperature correction unit, and the control unit; and an electrode unit configured to include the electrode and the temperature sensor and be detachably connected to the main body, in which the correction information may include first correction information on an individual characteristic of a component related to the temperature measurement in the electrode unit.

According to this, it is possible to facilitate replacement of the electrode disposed at the treatment target and to cancel an error caused by the individual difference of the component related to the temperature measurement in the replaced electrode unit. Therefore, it is possible to appropriately perform the treatment by measuring the treatment temperature with high accuracy and controlling the output of the high-frequency power.

In addition, in the high-frequency treatment device according to the aspect of the present invention, in which the correction information may include second correction information on an individual characteristic of a component related to the temperature measurement in the main body.

According to this, since it is possible to cancel an error caused by the individual difference of the component related to the temperature measurement in the main body, it is possible to appropriately perform the treatment, by measuring the treatment temperature with high accuracy and controlling the output of the high-frequency power.

Further, in the high-frequency treatment device according to the aspect of the present invention, the electrode unit may include a storage device configured to store the first correction information.

According to this, even in a case where the electrode unit is replaced, it is possible to easily acquire the first correction information, so that it is possible to easily handle the apparatus and easily perform the appropriate treatment.

Further, the high-frequency treatment device according to the aspect of the present invention, further includes: a main body configured to include the output unit, the temperature measurement unit, the temperature correction unit, and the control unit; and an electrode unit configured to include the electrode and the temperature sensor and be detachably connected to the main body, in which the correction information may include second correction information on an individual characteristic of a component related to the temperature measurement in the main body.

According to this, it is possible to facilitate replacement of the electrode disposed at the treatment target and to cancel an error caused by the individual difference of the component related to the temperature measurement in the main body. Therefore, it is possible to appropriately perform the treatment by measuring the treatment temperature with high accuracy and controlling the output of the high-frequency power.

In addition, in the high-frequency treatment device according to the aspect of the present invention, in which the correction information may include first correction information on an individual characteristic of a component related to the temperature measurement in the electrode unit.

According to this, since it is possible to cancel an error caused by the individual difference of the component related to the temperature measurement in the electrode unit, it is possible to appropriately perform the treatment, by measuring the treatment temperature with high accuracy and controlling the output of the high-frequency power.

Further, in the high-frequency treatment device according to the aspect of the present invention, the main body may include a correction information generation unit configured to generate the second correction information.

According to this, since it is possible to facilitate the generation of the second correction information, it is possible to reduce a manufacturing cost of the main body and to easily correct the second correction information according to an inter-annual variation or the like.

According to a high-frequency treatment device and a high-frequency treatment method according to the present invention, it is possible to achieve an excellent effect in that a treatment is appropriately performed by measuring a treatment temperature with high accuracy.

### Brief Description of Drawings

FIG. 1 is a schematic view illustrating an external appearance of a high-frequency treatment device according to a first embodiment of the present invention.
FIG. 2 is a block diagram illustrating an internal configuration of the high-frequency treatment device according to the first embodiment.
FIG. 3 is a block diagram illustrating a configuration related to temperature measurement.
FIGS. 4A and 4B are schematic views illustrating an operation state in a quadpolar manner.
FIG. 5 is a block diagram illustrating an internal configuration of a high-frequency treatment device according to a second embodiment.
FIGS. 6A to 6C are schematic views illustrating an operation state in a tripolar manner.

### Description of Embodiments

Hereinafter, embodiments of the present invention will be described with reference to the accompanying drawings.

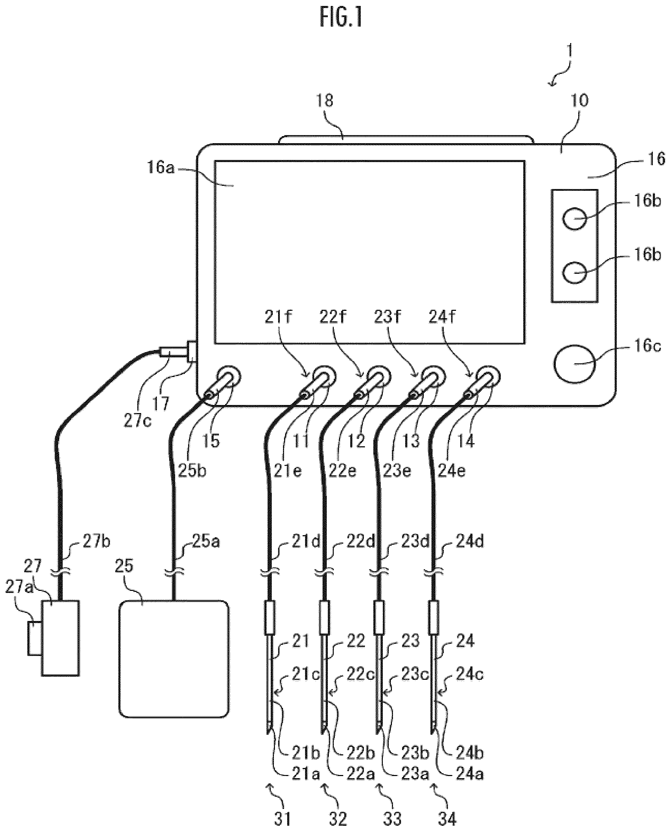

FIG. 1 is a schematic view illustrating an external appearance of a high-frequency treatment device 1 according to a first embodiment of the present invention. The high-frequency treatment device 1 of the present embodiment is for performing nerve blocking by partially heating a peripheral nerve with a high-frequency current. As illustrated in FIG. 1, the high-frequency treatment device 1 includes a main body 10, four electrodes 21, 22, 23, and 24 connected to the main body 10, a counter electrode plate 25 connected to the main body 10, and an operation switch 27 connected to the main body 10.

The main body 10 accommodates or supports an internal configuration, which will be described below. On a front surface of the main body 10, four main body-side connectors 11 to 14 to which the electrodes 21 to 24 are respectively connected and a main body-side connector 15 to which the counter electrode plate 25 is connected are provided at a lower portion. An operation unit 16 that accepts an operation of a user is further provided on the front surface of the main body 10. The operation unit 16 is configured with a touch panel display 16a that accepts input operations such as various settings and displays various types of information, a button 16b that receives start and end operations of a treatment, and a control knob 16c for adjusting an output power.

A main body-side connector 17 to which an operation switch 27 is connected is provided on a left side surface of the main body 10. Further, a handle 18 for carrying the main body 10 is provided on an upper portion of the main body 10. Although not illustrated, a rear surface of the main body 10 is provided with a power connector and a power switch which are connected to a commercial alternating current power supply to receive a supply of power.

The electrodes 21 to 24 are inserted into a body of a patient as a treatment target on which a high-frequency treatment is to be performed, and are used to apply a high-frequency current into the body. In the present embodiment, the electrodes 21 to 24 are configured in a needle tubular shape capable of piercing a human body or the like and injecting a drug or the like via the electrodes 21 to 24. The electrodes 21 to 24 have insulating portions 21b to 24b mostly coated with insulation except for non-insulating portions 21a to 24a at tip portions, and the high-frequency current is output from the non-insulating portions 21a to 24a.

Further, thermocouples 21c to 24c, which are temperature sensors for measuring a treatment temperature, are disposed inside the electrodes 21 to 24. The electrodes 21 to 24 and the thermocouples 21c to 24c are electrically connected to electrode-side connectors 21e to 24e via electrode cables 21d to 24d, and the electrode-side connectors 21e to 24e are connected to the main body-side connectors 11 to 14 to be electrically connected to the main body 10.

That is, electrode units 31 to 34 detachably connected to the main body 10 are configured with the electrodes 21 to 24, the thermocouples 21c to 24c, the electrode cables 21d to 24d, and the electrode-side connectors 21e to 24e, respectively. In this manner, with the configuration in which the electrodes 21 to 24 to be inserted into a human body or the like are detachable from the main body 10 as the electrode units 31 to 34, not only sterilization or disinfection are facilitated, but also it is possible to facilitate replacement of the electrode units 31 to 34.

Storage devices 21f to 24f having a known configuration such as a flash memory are disposed in the electrode-side connectors 21e to 24e. The storage devices 21f to 24f store first correction information Ke on individual characteristics of components related to temperature measurement in the electrode units 31 to 34, such as the thermocouples 21c to 24c and a compensation lead wire.

In the present embodiment, it is possible to measure a treatment temperature with high accuracy by correcting a temperature measurement value measured by the thermocouples 21c to 24c using the first correction information Ke. The storage devices 21f to 24f are electrically connected to the main body 10 by connecting the electrode-side connectors 21e to 24e to the main body-side connectors 11 to 14. Therefore, even in a case where the electrode units 31 to 34 are replaced, the internal configuration of the main body 10 makes it possible to easily acquire the first correction information Ke.

The electrodes 21 to 24 may have other shapes, and may be rod-shaped, for example, inserted into a needle tube or a catheter. Further, the thermocouple 21c to 24c may be provided separately from the electrodes 21 to 24 and may be disposed in the vicinity of the electrodes 21 to 24 to be inserted into a human body or the like. In addition, the storage devices 21f to 24f may store various types of information such as an identification number of the electrodes 21 to 24, a manufacturing lot, and a use history, for example, together with the first correction information Ke. Further, the storage devices 21f to 24f may be disposed at a portion other than the electrode-side connectors 21e to 24e.

The counter electrode plate 25 is a flat plate-shaped electrode that is disposed to be attached on a skin surface of the patient as a treatment target, and is for passing the high-frequency current between the electrodes 21 to 24 inserted into the inside. That is, the counter electrode plate 25 is used in a case where the high-frequency current flows in a so-called monopolar manner. The counter electrode plate 25 is electrically connected to the main body 10 via a counter electrode plate cable 25a, a counter electrode plate-side connector 25b, and the main body-side connector 15. In the present embodiment, the counter electrode plate 25 is configured in a rectangular flat plate shape, and the counter electrode plate 25 may have another shape.

The operation switch 27 is provided to the patient as a treatment target during a treatment, and is operated by the patient. The operation switch 27 includes a push button 27a that is pressed by the patient, and is electrically connected to the main body 10 via an operation switch cable 27b, an operation switch-side connector 27c, and the main body-side connector 17.

In the present embodiment, in a case where the patient feels pain during the treatment, the patient presses the push button 27a to discriminate whether or not the patient feels the pain. Then, an output of the high-frequency power is reduced based on the fact that the operation switch 27 detects the operation of the patient, thereby reducing the pain of the patient.

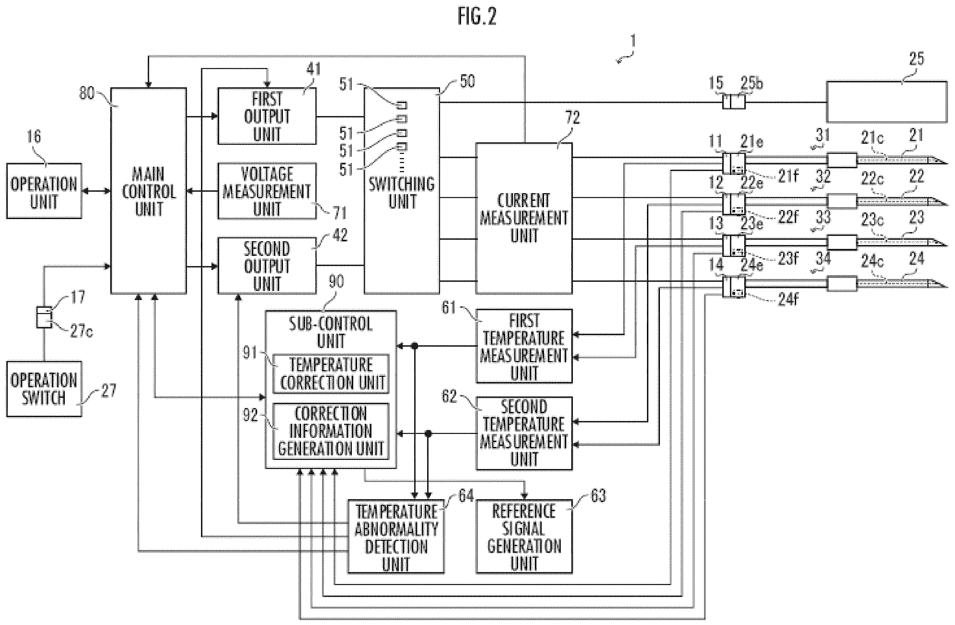

FIG. 2 is a block diagram illustrating an internal configuration of the high-frequency treatment device 1. As illustrated in FIG. 2, the high-frequency treatment device 1 includes, as the internal configuration, a first output unit 41 and a second output unit 42, a switching unit 50, a first temperature measurement unit 61 and a second temperature measurement unit 62, a reference signal generation unit 63, a temperature abnormality detection unit 64, a voltage measurement unit 71, a current measurement unit 72, a main control unit 80, and a sub-control unit 90.

Based on power supplied from the commercial alternating current power supply, the first output unit 41 and the second output unit 42 generate and output high-frequency power with a preset frequency (for example, 470 to 490 kHz) and a voltage based on an output control signal from the main control unit 80 (for example, 18 to 22 Vrms). Each of the first output unit 41 and the second output unit 42 is configured with a known circuit having a transformer, whereby the body as a treatment target is insulated from the commercial alternating current power supply.

The first output unit 41 is connected to the main body-side connector 11, the main body-side connector 12, and the main body-side connector 13 and the main body-side connector 15 via the switching unit 50, that is, is connected to be capable of outputting the high-frequency power to the electrode 21, the electrode 22, and the electrode 23 and the counter electrode plate 25. In addition, the second output unit 42 is connected to the main body-side connector 13, the main body-side connector 14, and the main body-side connector 15 via the switching unit 50, that is, is connected so as to be capable of outputting the high-frequency power to the electrode 23, the electrode 24, and the counter electrode plate 25.

Therefore, an output unit of the present invention is configured with the first output unit 41 and the second output unit 42. In the present embodiment, by providing two output units of the first output unit 41 and the second output unit 42, it is possible to pass the high-frequency current while simultaneously performing output control in a stable manner with two electrode sets (for example, an electrode set of the electrode 21 and the electrode 22 and an electrode set of the electrode 23 and the electrode 24).

The switching unit 50 operates under control of the sub-control unit 90, and switches the connection between the first output unit 41 and the second output unit 42, and the main body-side connectors 11 to 15. That is, the switching unit 50 switches to which electrode set of electrode sets, in which at least two of the electrodes 21 to 24 and the counter electrode plate 25 are combined, the high-frequency current flows.

The switching unit 50 is configured with a circuit including a plurality of switches 51 including a semiconductor switch such as a metal oxide semiconductor field effect transistor (MOSFET) or a reed relay, and is provided between the first output unit 41 and the second output unit 42, and the electrodes 21 to 24 and the counter electrode plates 25.

In the present embodiment, since the two output units of the first output unit 41 and the second output unit 42 are provided, by reducing the number of switches 51 and simplifying the configuration of the switching unit 50, it is possible to speed up switching of connection between the first output unit 41 and the second output unit 42, and each of the electrodes 21 to 24.

The first temperature measurement unit 61 and the second temperature measurement unit 62 are configured with a known circuit or the like, generate a temperature measurement signal (for example, 25 mV/°C) based on signals (voltage by thermoelectromotive power) received from the thermocouples 21c to 24c (temperature measurement process), and transmits the temperature measurement signal to the sub-control unit 90 and the temperature abnormality detection unit 64. The first temperature measurement unit 61 is connected to the main body-side connectors 11 and 13, and the second temperature measurement unit 62 is connected to the main body-side connectors 12 and 14. That is, the first temperature measurement unit 61 is connected to the thermocouple 21c incorporated in the electrode 21 and the thermocouple 23c incorporated in the electrode 23. In addition, the second temperature measurement unit 62 is connected to the thermocouple 22c incorporated in the electrode 22 and the thermocouple 24c incorporated in the electrode 24.

In the present embodiment, by providing two temperature measurement units of the first temperature measurement unit 61 and the second temperature measurement unit 62, it is possible to accurately measure ambient temperatures of all the four electrodes 21 to 24 in a short time. That is, while ensuring a sufficient measurement time for each of the thermocouples 21c to 24c, the entire measurement time is not lengthened.

In addition, the first temperature measurement unit 61 and the second temperature measurement unit 62 respectively measure each of both ambient temperatures of the electrodes 21, 22, or 23 from which a high-frequency power is output from the first output unit 41 and ambient temperatures of the electrodes 23 and 24 from which a high-frequency power is output from the second output unit 42, so that even in a case where either the first temperature measurement unit 61 or the second temperature measurement unit 62 fails, occurrence of a temperature abnormality by the first output unit 41 or the second output unit 42 is detectable, thereby improving safety.

The reference signal generation unit 63 is configured with a known circuit or the like, and generates a temperature reference signal serving as a reference for determining whether or not the temperature measured by the first temperature measurement unit 61 or the second temperature measurement unit 62 is an abnormal temperature. The reference signal generation unit 63 is controlled by the sub-control unit 90 to generate, for example, the temperature reference signal corresponding to a temperature setting value of +7°C. Here, the temperature setting value is input to the operation unit 16 as a treatment temperature maintained during the treatment, acquired by the main control unit 80, and transmitted to the sub-control unit 90. The generated temperature reference signal is transmitted to the temperature abnormality detection unit 64.

The temperature abnormality detection unit 64 is configured with a known circuit or the like, and compares a temperature reference signal received from the reference signal generation unit 63 with a temperature measurement signal received from the first temperature measurement unit 61 or the second temperature measurement unit 62 and detects the occurrence of the temperature abnormality. In a case where a voltage of the temperature measurement signal is higher than a voltage of the temperature reference signal, the temperature abnormality detection unit 64 transmits an output stop signal to the first output unit 41, the second output unit 42, and the main control unit 80. Upon receiving the output stop signal, the first output unit 41 and the second output unit 42 stop the output of the high-frequency power. In addition, the main control unit 80 that receives the output stop signal performs a temperature abnormality process such as an alarm display.

The voltage measurement unit 71 is configured with a known circuit or the like, and individually measures a voltage of the high-frequency power output by the first output unit 41 and the second output unit 42. The voltage measurement unit 71 generates a voltage measurement signal based on a high-frequency voltage generated by the first output unit 41 and the second output unit 42, and transmits the voltage measurement signal to the main control unit 80.

The current measurement unit 72 is configured with a known circuit or the like, and individually measures a high-frequency current flowing through the electrodes 21 to 24. The current measurement unit 72 generates a current measurement signal based on the high-frequency current flowing through the electrodes 21 to 24, and transmits the current measurement signal to the main control unit 80.

The main control unit 80 includes a known configuration of a CPU, a ROM, a RAM, and the like, and controls each unit of the high-frequency treatment device 1 such as the operation unit 16, the first output unit 41 and the second output unit 42, or the like to execute the high-frequency treatment. Based on the input operation accepted by the operation unit 16, the main control unit 80 transmits a connection mode signal indicating a connection mode of the first output unit 41 and the second output unit 42, and the main body-side connectors 11 to 15 to the sub-control unit 90.

The main control unit 80 also starts or ends the output of the high-frequency power from the first output unit 41 or the second output unit 42 based on the input operation accepted by the operation unit 16, and also performs the output control of the high-frequency power with PID control so that a temperature measurement value received from the sub-control unit 90 is substantially equal to a control target value (output control process). That is, a control unit of the present invention is configured with the main control unit 80.

As the output control, the main control unit 80 performs two types of control of normal output control and output reduction control in which the output of the high-frequency power is reduced than the normal output control. In the normal output control, the main control unit 80 sets the control target value to the temperature setting value (first target value) input to the operation unit 16, and performs the output control. Further, in the output reduction control, the main control unit 80 performs the control by setting the control target value to a second target value, which is a value lower than the current temperature measurement value by 10°C.

The output reduction control is performed to reduce pain felt by the patient as a treatment target. Therefore, in a case where the operation switch 27 detects an operation of the patient, the main control unit 80 shifts the output control from the normal output control up to that point to the output reduction control. The main control unit 80 also shifts the output control from the output reduction control to the normal output control when the operation switch 27 does not detect an operation of the patient (when the patient stops the operation of the operation switch 27).

Further, the main control unit 80 calculates a voltage measurement value, a current measurement value, a power measurement value, and an impedance measurement value, based on the voltage measurement signal received from the voltage measurement unit 71 and the current measurement signal received from the current measurement unit 72, and displays the voltage measurement value, the current measurement value, the power measurement value, and the impedance measurement value on the touch panel display 16a together with the temperature measurement value.

The main control unit 80 uses an output voltage as an operation amount in the high-frequency thermal coagulation method and an output pulse width as an operation amount in the pulse high-frequency method. Further, in a case where an output voltage value is set by an input operation accepted by the operation unit 16, the main control unit 80 performs the output control of the high-frequency power with PID control so that the voltage measurement value becomes equal to a voltage setting value.

In the same manner as the main control unit 80, the sub-control unit 90 has a known configuration of a CPU, a ROM, a RAM, and the like, and controls the switching unit 50, the first temperature measurement unit 61, the second temperature measurement unit 62, and the reference signal generation unit 63. The sub-control unit 90 controls ON and OFF of the plurality of switches 51 included in the switching unit 50 based on a connection mode signal transmitted from the main control unit 80, and switches connection states of the first output unit 41 and the second output unit 42, the main body-side connectors 11 to 15.

The sub-control unit 90 also calculates a temperature measurement value based on the temperature measurement signal received from the first temperature measurement unit 61 or the second temperature measurement unit 62, and transmits the temperature measurement value to the main control unit 80 (temperature correction process). Further, the sub-control unit 90 controls the reference signal generation unit 63 based on the temperature setting value received from the main control unit 80 to generate a temperature reference signal, and transmits the temperature reference signal to the temperature abnormality detection unit 64.

In addition, the sub-control unit 90 includes a temperature correction unit 91 as a functional configuration realized by a CPU executing a program. The temperature correction unit 91 corrects the received temperature measurement signal, in the calculation of the temperature measurement value. This correction is performed based on second correction information Km stored in a storage device (not illustrated) such as a flash memory included in the sub-control unit 90, together with the first correction information Ke stored in the storage devices 21f to 24f described above.

The first correction information Ke is information on individual characteristics of components related to temperature measurement in the electrode units 31 to 34 as described above. In the present embodiment, nine correction reference temperatures (5°C, 15°C, 25°C, 35°C, 45°C, 55°C, 65°C, 75°C, and 85°C) are set every 10°C in a range of 5°C to 85°C, and the first correction information Ke is set for each correction reference temperature. Therefore, the nine pieces of first correction information Ke are stored in the storage devices 21f to 24f of the electrode units 31 to 34, respectively. In this manner, by setting the first correction information Ke for each correction reference temperature, it is possible to perform correction precisely corresponding to characteristics of the thermocouple 21c and the like.

The second correction information Km is information on individual characteristics of components related to temperature measurement (mainly components in the first temperature measurement unit 61 and the second temperature measurement unit 62) in the main body 10. In the present embodiment, each second correction information Km is set for the first temperature measurement unit 61 and the second temperature measurement unit 62 one by one. Therefore, the two pieces of first correction information Km are stored in the storage device included in the sub-control unit 90. In this manner, by setting the second correction information Km one by one, it is possible to facilitate the generation of the second correction information Km.

In the present embodiment, by correcting the temperature measurement signal by using the second correction information Km related to the main body 10 together with the first correction information Ke related to the electrode units 31 to 32, it is possible to measure the treatment temperature with higher accuracy. Further, in the present embodiment, the sub-control unit 90 generates the second correction information Km. Therefore, the sub-control unit 90 includes a correction information generation unit 92 as a functional configuration realized by a CPU executing a program.

The switching unit 50, the first temperature measurement unit 61, the second temperature measurement unit 62, the reference signal generation unit 63, the temperature abnormality detection unit 64, the voltage measurement unit 71, the current measurement unit 72, and the sub-control unit 90 are insulated from the commercial alternating current power supply, in order to protect the body as a treatment target from a high-voltage current.

Next, a method of calculating a temperature measurement value will be described.

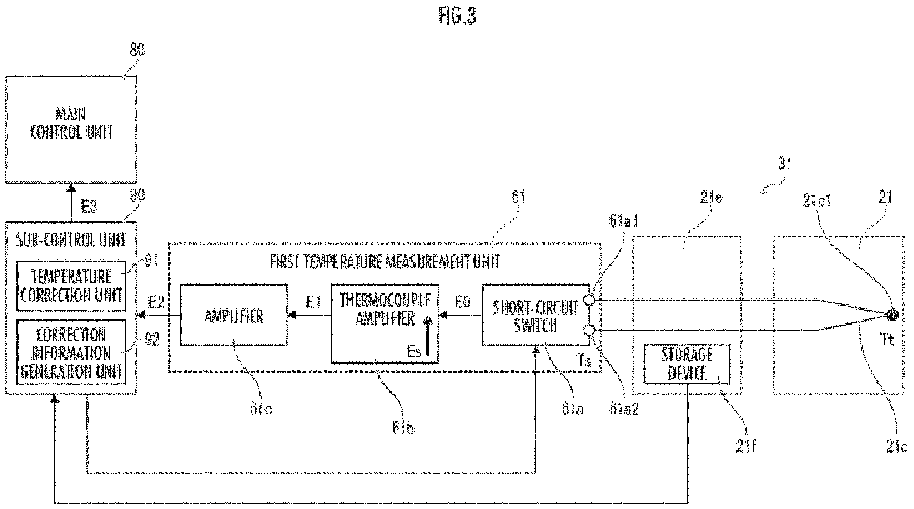

FIG. 3 is a block diagram illustrating a configuration related to temperature measurement. FIG. 3 illustrates the first temperature measurement unit 61 and the electrode unit 31 as an example, and the second temperature measurement unit 62 and the electrode units 32 to 33 also have the same configuration.

As illustrated in FIG. 3, the first temperature measurement unit 61 includes a short-circuit switch 61a to which the thermocouple 21c is connected, a thermocouple amplifier 61b connected to the short-circuit switch 61a, and an amplifier 61c connected to the thermocouple amplifier 61b.

The short-circuit switch 61a short-circuits input terminals 61a1 and 61a2 serving as reference contacts (cold contacts). The thermocouple amplifier 61b outputs a first amplification voltage E1 obtained by amplifying a thermoelectromotive voltage of the thermocouple 21c corresponding to a temperature (measurement temperature) Tm at a temperature measurement contact 21c1 with a predetermined gain.

The thermocouple amplifier 61b includes a temperature sensor (not illustrated) that measures a temperature (reference contact temperature) Ts of the input terminals 61a1 and 61a2, and an arithmetic device (not illustrated) that calculates a thermoelectromotive voltage Es corresponding to the measured reference contact temperature Ts based on a reference thermoelectromotive power table to perform a reference contact compensation.

Therefore, the first amplification voltage E1 output by the thermocouple amplifier 61b is a voltage obtained by amplifying a voltage obtained by adding the calculated thermoelectromotive voltage Es corresponding to the reference contact temperature Ts to an input thermoelectromotive voltage E0 (thermoelectromotive voltage corresponding to a temperature difference between the measurement temperature Tm and the reference contact temperature Ts) of the thermocouple 21c, when the short-circuit switch 61a is in an open state. Further, when the short-circuit switch 61a is in a short-circuit state, the thermoelectromotive voltage E0 of the thermocouple 21c is 0. Therefore, the first amplification voltage E1 output by the thermocouple amplifier 61b is a voltage obtained by amplifying the calculated thermoelectromotive voltage Es corresponding to the reference contact temperature Ts.

The amplifier 61c generates a second amplification voltage E2 obtained by amplifying the input first amplification voltage E1 to, for example, 25 mV/°C, and transmits the second amplification voltage E2 as a temperature measurement signal to the sub-control unit 90. In the present embodiment, the amplifier 61c is provided to increase a level of the temperature measurement signal and increase a resolution, so that accuracy of the calculation of the temperature measurement value is improved.

The temperature correction unit 91 of the sub-control unit 90 calculates a temperature measurement value by correcting the input second amplification voltage E2 based on the first correction information Ke and the second correction information Km. The temperature correction unit 91 acquires the first correction information Ke from the storage devices 21f to 24f at a predetermined timing such as when the electrode units 31 to 34 are connected to the main body 10 or when a treatment is started, and stores the first correction information Ke in the RAM or the like of the sub-control unit 90. In the same manner, at a predetermined timing, the temperature correction unit 91 acquires the second correction information Km from the storage device of the sub-control unit 90, and stores the second correction information Km in the RAM or the like of the sub-control unit 90.

In the calculation of the temperature measurement value by the thermocouple 21c, the temperature correction unit 91 first controls the short-circuit switch 61a to short-circuit the input terminals 61a1 and 61a2, and acquires a reference second amplification voltage E2s corresponding to the reference contact temperature Ts. Next, the temperature correction unit 91 controls the short-circuit switch 61a to release the input terminals 61a1 and 61a2, and acquires a temperature measurement second amplification voltage E2m corresponding to the measurement temperature Tm.

Next, the temperature correction unit 91 subtracts the reference second amplification voltage E2s from the temperature measurement second amplification voltage E2m to calculate a difference voltage ΔE2 (= E2m-E2s). Then, a difference temperature Td (= Tm-Ts) obtained by converting the difference voltage ΔE2 into a temperature is calculated.

Next, the temperature correction unit 91 calculates a correction difference voltage ΔE2c (= Ke × ΔE2) by multiplying the difference voltage ΔE2 by the first correction information Ke of a correction reference temperature closest to the difference temperature Td.

Next, the temperature correction unit 91 calculates a primary correction second amplification voltage E2c1 (= ΔE2c + E2s) by adding the reference second amplification voltage E2s to the correction difference voltage ΔE2c.

Next, the temperature correction unit 91 multiplies the second correction information Km related to the first temperature measurement unit 61 by a primary correction second amplification voltage E2c to calculate a secondary correction second amplification voltage E2c2 (= Ke × E2c1).

Next, the temperature correction unit 91 generates a correction temperature signal based on the secondary correction second amplification voltage E2c2. This correction temperature signal may be, for example, a voltage E3 (= E2c2) equal to the secondary correction second amplification voltage E2c2, or may be a binary signal indicating a temperature conversion value (that is, temperature measurement value) of the secondary correction second amplification voltage E2c2.

The sub-control unit 90 transmits the generated correction temperature signal to the main control unit 80. The main control unit 80 controls the output of the first output unit 41 and the second output unit 42 so that the temperature measurement value indicated by the received correction temperature signal is equal to the control target value.

In this manner, by performing the correction using the first correction information Ke, it is possible to correct the measurement error caused by the individual difference of each component included in the electrode units 31 to 34. Therefore, it is possible to perform the temperature measurement with high accuracy. Further, by performing the correction using the second correction information Km, it is possible to correct the measurement error caused by the individual difference of each component included in the main body 10, and thus it is possible to perform the temperature measurement with higher accuracy.

In the present embodiment, the correction is performed by using both the first correction information Ke and the second correction information Km, and depending on an accuracy degree of each component included in the electrode unit 31 and each component included in the main body 10, the correction may be performed by using only any one of the first correction information Ke and the second correction information Km.

Further, in the calculation of the correction difference voltage ΔE2c, instead of multiplying the difference voltage ΔE2 by the first correction information Ke of a correction reference temperature closest to the difference temperature Td, the first correction information Ke corresponding to the difference temperature Td may be calculated by interpolating the correction reference temperature and multiplied.

Next, a method of generating the first correction information Ke will be described.

The first correction information Ke of the present embodiment is an error rate from an ideal voltage Ei based on reference thermoelectromotive power of the voltage output from the electrode units 31 to 34 to the main body 10 as the thermoelectromotive voltage E0 of the thermocouples 21c to 24c, and is generated for each of the electrode units 31 to 34.

In the generation of the first correction information Ke, a practitioner first prepares a temperature calibrator. Then, after the electrodes 21 to 24 of the electrode units 31 to 34 are inserted into the temperature calibrator, a temperature of the temperature calibrator is set to room temperature + each correction reference temperature (room temperature + 5°C, room temperature + 15°C, room temperature + 25°C, room temperature + 35°C, room temperature + 45°C, room temperature + 55°C, room temperature + 65°C, room temperature + 75°C, and room temperature + 85°C), and an output voltage Em from a terminal of the electrode-side connectors 21e to 24e is measured for each correction reference temperature.

Next, the practitioner divides the measured output voltage Em by a reference thermoelectromotive power Ei at a correction reference temperature to calculate the first correction information Ke (= Em / Ei) for each correction reference temperature. The calculated first correction information Ke is stored in the storage devices 21f to 24f of the electrode units 31 to 34.

By generating the first correction information Ke in this manner, as an error rate caused not only by the individual difference of the thermocouple 21c to 24c but also by the individual difference of all the components related to the temperature measurement in the electrode units 31 to 34, it is possible to generate the first correction information Ke. Further, an error rate caused by an assembly error of the electrode units 31 to 34, such as a disposition position of the thermocouple 21c to 24c in the electrodes 21 to 24, is also included in the first correction information Ke, and thus it is possible to perform the correction with higher accuracy.

The first correction information Ke is generated and stored in the storage devices 21f to 24f by a manufacturer when the high-frequency treatment device 1 is manufactured or when the electrode units 31 to 34 as replacement components are manufactured, and also can be generated and stored by a person other than the manufacturer, such as a user of the high-frequency treatment device 1, for example.

Next, a method of generating the second correction information Km will be described.

The second correction information Km of the present embodiment is an error rate from an optimal second amplification voltage (ideal second amplification voltage) E2i of the second amplification voltage E2 input to the sub-control unit 90. In the present embodiment as described above, the sub-control unit 90 includes the correction information generation unit 92, and the second correction information Km is generated by using the main body 10.

In the generation of the second correction information Km, the practitioner first prepares a calibrated thermocouple and a calibrated constant-temperature water tank. The thermocouple is connected to the main body 10 via, for example, the main body-side connector 11, and then put into the constant-temperature water tank set to a predetermined low-temperature-side setting temperature Ta (for example, 41°C). The correction information generation unit 92 is activated based on a predetermined input operation by the practitioner, and stores the low-temperature-side second amplification voltage E2a corresponding to the low-temperature-side setting temperature Ta output from the first temperature measurement unit 61 in a RAM or the like.

Next, the practitioner puts the thermocouple into the constant-temperature water tank set to a predetermined high-temperature-side setting temperature Tb (for example, 80°C). The correction information generation unit 92 is activated based on a predetermined input operation by the practitioner, and stores the high-temperature-side second amplification voltage E2b corresponding to the high-temperature-side setting temperature Tb output from the first temperature measurement unit 61 in the RAM or the like.

When both the low-temperature-side second amplification voltage E2a and the high-temperature-side second amplification voltage E2b are stored, the correction information generation unit 92 calculates the second correction information Km (= (E2b - E2a) / (Tb - Ta)), by dividing a difference obtained by subtracting the low-temperature-side second amplification voltage E2a from the high-temperature-side second amplification voltage E2b by a difference obtained by subtracting the low-temperature-side setting temperature Ta from the high-temperature-side setting temperature Tb, automatically or based on a predetermined input operation by the practitioner. In addition, the correction information generation unit 92 stores the calculated second correction information Km in the storage device including the sub-control unit 90.

Next, the practitioner connects the thermocouple to the main body 10 via, for example, the main body-side connector 12, causes the correction information generation unit 92 to generate the second correction information Km for the second temperature measurement unit 62 in the same procedure, and stores the second correction information Km in the storage device included in the sub-control unit 90.

The second correction information Km is mainly related to characteristics of components (thermocouple amplifier 61b and amplifier 61c) that amplify signals in the first temperature measurement unit 61 and the second temperature measurement unit 62. In a case where the input thermoelectromotive voltage E0 is small, an error due to individual differences is considered to be small, so that it is possible to easily and simply generate the second correction information Km, by adopting the method described above.

In a case where it is necessary to consider the characteristics of the main body-side connectors 11 to 14 and the characteristics of components and the like provided between the main body-side connectors 11 to 14, and the first temperature measurement unit 61 and the second temperature measurement unit 62, the second correction information Km may be generated for each of the main body-side connectors 11 to 14. Further, the generation of the second correction information Km and the storage in the storage device included in the sub-control unit 90 are performed by the manufacturer when the high-frequency treatment device 1 is manufactured, and can also be performed by a person other than the manufacturer.

By providing the correction information generation unit 92 in the main body 10, it is possible to reduce a manufacturing cost of the main body 10. Further, unlike the electrode units 31 to 34, the main body 10 is used for a relatively long period. Therefore, by providing the main body 10 with the correction information generation unit 92, it is possible to easily correct the second correction information Km according to an inter-annual variation or the like of the components in the main body 10.

Next, an operation of the high-frequency treatment device 1 will be described.

The high-frequency treatment device 1 is capable of four types of output formats of a high-frequency current to a treatment target: monopolar, bipolar, tripolar, and quadpolar. In the present embodiment, the monopolar manner has an output format in which a high-frequency current flows through an electrode set in which any one of the electrodes 21 to 24 and the counter electrode plate 25 are combined, and the bipolar manner has an output format in which a high-frequency current flows by using the electrodes 21 and 22 or the electrodes 23 and the electrode 24 as an electrode set.

In addition, the tripolar manner has an output format in which a high-frequency current flows between the electrode 21, and the electrode 22 and the electrode 23, between the electrode 22, and the electrode 21 and the electrode 23, or between the electrode 23, and the electrode 21 and the electrode 22, by using the electrode 21, the electrode 22, and the electrode 23 as an electrode set. Further, in the tripolar manner, it is also possible to switch a state in which a high-frequency current flows between the electrode 21, and the electrode 22 and the electrode 23, a state in which a high-frequency current flows between the electrode 22, and the electrode 21 and the electrode 23, and a state in which a high-frequency current flows between the electrode 23, and the electrode 21 and the electrode 22, at a specific cycle (for example, 0.1 seconds).

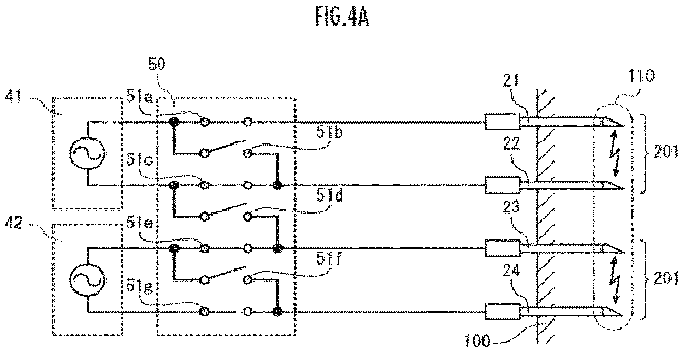

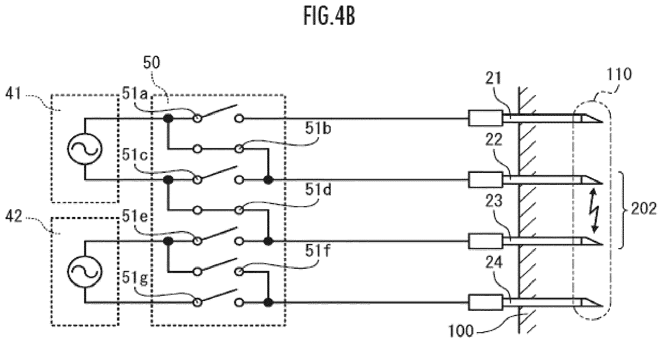

Hereinafter, the operation of the high-frequency treatment device 1 will be described by using a case where an output format is quadpolar, as an example. FIGS. 4A and 4B are schematic views illustrating an operation state in a quadpolar manner. In order to facilitate understanding, FIGS. 4A and 4B describe only switches 51a to 51g necessary for description among the plurality of switches 51 included in the switching unit 50.

As illustrated in FIGS. 4A and 4B, in a case where a high-frequency treatment in a quadpolar manner is performed, first, all the four electrodes 21 to 24 are inserted into a treatment target 100. At this time, the electrodes 21 to 24 are arranged in a row at substantially equal intervals, for example, in this order.

When the electrodes 21 to 24 are arranged, nerve exploration, measurement of an impedance of the peripheral tissue of the electrodes 21 to 24, and the like are performed. Meanwhile, since the nerve exploration and the measurement are techniques in the related art, the description thereof will be omitted. In addition, before or after the insertion of the electrodes 21 to 24, an anesthetic is injected in the vicinity of disposition positions of the electrodes 21 to 24, and local anesthesia is performed to reduce pain due to overheating during the treatment.

When the electrodes 21 to 24 are appropriately arranged and the operation unit 16 accepts a quadpolar manner selection operation by a user such as a doctor, the main control unit 80 transmits a connection mode signal indicating a quadpolar connection mode to the sub-control unit 90. When receiving the connection mode signal indicating the quadpolar connection mode from the main control unit 80, the sub-control unit 90 controls the switching unit 50, and sets a connection state of the first output unit 41 and the second output unit 42, and the electrodes 21 to 24 to be a first state illustrated in FIG. 4A.

Specifically, in the first state, the sub-control unit 90 turns on the switch 51a, the switch 51c, the switch 51e, and the switch 51g, and turns off the switch 51b, the switch 51d, and the switch 51f. Accordingly, the first output unit 41 is connected to the electrode 21 and the electrode 22, and the second output unit 42 is connected to the electrode 23 and the electrode 24. Therefore, a state in which a high-frequency current by high-frequency power output by the first output unit 41 flows between the electrodes 21 and 22, and a high-frequency current by the high-frequency power output by the second output unit 42 flows between the electrodes 23 and 24 is obtained. As a result, a first electrode set 201, which is an electrode set through which a high-frequency current flows in the first state, is configured with the electrodes 21 and 22, and the electrodes 23 and 24, respectively.

After that, when the operation unit 16 accepts a start operation of a treatment after the operation unit 16 receives an operation of selecting the high-frequency thermal coagulation method or the pulse high-frequency method and an input operation of a temperature setting value (for example, 80°C) and a treatment time (for example, 3 minutes) by the user (in a case where the pulse high-frequency method is selected, the main control unit 80 sets the temperature setting value to 42°C, so that only the treatment time is input), the main control unit 80 controls the first output unit 41 and the second output unit 42 to start an output of high-frequency power. In addition, the main control unit 80 transmits an output start signal indicating that the output is started to the sub-control unit 90.

When the output start signal is received from the main control unit 80, the sub-control unit 90 controls the switching unit 50 so that the connection state of the first output unit 41 and the second output unit 42, and the electrodes 21 to 24 is switched between the first state illustrated in FIG. 4A in a specific cycle (in the present embodiment, 0.1 seconds) and a second state illustrated in FIG. 4B.

In the second state, the sub-control unit 90 turns on the switch 51b and the switch 51d, and turns off the switch 51a, the switch 51c, the switch 51e, the switch 51f, and the switch 51g, as illustrated in FIG. 4B. Accordingly, a state is obtained in which the first output unit 41 is connected to the electrode 22 and the electrode 23, and a high-frequency current by the high-frequency power output by the first output unit 41 flows between the electrode 22 and the electrode 23. As a result, a second electrode set 202, which is an electrode set through which a high-frequency current flows in the second state, is configured with the electrode 22 and the electrode 23.

During the output of the high-frequency power, with switching control of the switching unit 50 by the sub-control unit 90, the first state in which a high-frequency current flows between the electrodes 21 and 22 and between the electrodes 23 and 24 and the second state in which a high-frequency current flows between the electrodes 22 and 23 are periodically switched. Accordingly, each of a region between the electrodes 21 and 22 in the periphery of the electrodes 21 and 22, a region between the electrodes 22 and 23 in the periphery of the electrodes 22 and 23, and a region between the electrodes 23 and 24 in the periphery of the electrodes 23 and 24 is heated intermittently. Meanwhile, in the present embodiment, the switching cycle is set to 0.1 seconds, so that a temperature of each region does not drop too much when not being heated.

The temperature correction unit 91 of the sub-control unit 90 corrects a temperature measurement signal received from the first temperature measurement unit 61 or the second temperature measurement unit 62 in parallel with the switching control of the switching unit 50 to generate a correction temperature signal and transmits the correction temperature signal to the main control unit 80. The main control unit 80 performs the output control of the first output unit 41 and the second output unit 42 based on the received correction temperature signal.

In the output control, after a temperature measurement value indicated by the correction temperature signal received from the sub-control unit 90 is increased up to a temperature substantially equal to a control target value, the main control unit 80 controls the output of the first output unit 41 and the second output unit 42 so as to maintain the state in which the temperature measurement value is substantially equal to the control target value Accordingly, a region 110 between the electrodes 21 to 24 in the periphery of the electrodes 21 to 24 is maintained at a temperature substantially equal to the temperature setting value as a whole, during the output of the high-frequency power, and thermal coagulation or degeneration of the tissue occurs.

The main control unit 80 changes the control target value according to the presence or absence of detection of an operation by the operation switch 27, and switches between the normal output control and the output reduction control. The output reduction control is mainly performed in a case where the effect of anesthesia is not sufficiently exhibited in an initial stage of the treatment.

When a treatment time input in advance elapses after the start of the output of the high-frequency power, the main control unit 80 stops the output of the high-frequency power from the first output unit 41 and the second output unit 42. The main control unit 80 also transmits an output end signal to the sub-control unit 90. The sub-control unit 90 that receives the output end signal from the main control unit 80 stops switching control between the first state and the second state with respect to the switching unit 50. With the above, one treatment is completed. A nerve tissue contained in a region 110 is heated at a temperature of, for example, 80°C for 3 minutes, and as a result, a pain signal is blocked.

With the high-frequency treatment device 1, by correcting the temperature measurement signal by the first correction information Ke, it is possible to cancel an error caused by individual differences of the components related to the temperature measurement in the electrode unit 31. Further, by correcting the temperature measurement signal by the second correction information Km, it is possible to cancel an error caused by individual differences of the components related to the temperature measurement in the main body 10.

Accordingly, it is possible to measure an ambient temperature of the electrodes 21 to 24 with high accuracy and control the output of the high-frequency power. Therefore, it is possible to accurately maintain a preset treatment temperature for heating the nerve tissue and appropriately perform the treatment. In other words, it is possible to prevent the thermal coagulation or degeneration of the tissue from becoming insufficient or excessive due to the nerve tissue being heated at a temperature deviating from the preset treatment temperature.

The first correction information Ke and the second correction information Km may be stored in, for example, another storage device provided in the main body 10, or may be stored in an external storage device connected to the main body 10. Further, the first correction information Ke and the second correction information Km may be stored by the sub-control unit 90 or the main control unit 80 by, for example, an input operation by the user. In this case, the correction information generation unit 92 may be omitted.

Further, the temperature correction unit 91 may be provided in the main control unit 80. In this case, the temperature correction unit 91 corrects a temperature measurement signal received from the first temperature measurement unit 61 or the second temperature measurement unit 62, or the sub-control unit 90 to calculate a temperature measurement value, and the main control unit 80 performs the output control based on the calculated temperature measurement value. In the same manner, the correction information generation unit 92 may be included in the main control unit 80. Further, the temperature correction unit 91 and the correction information generation unit 92 may be provided separately from the sub-control unit 90 or the main control unit 80.

Further, the electrode units 31 to 34 may include a temperature sensor other than the thermocouples 21c to 24c, such as a temperature measurement resistor, for example. Also in this case, it is possible to obtain the same effect by performing the correction using the first correction information Ke or the second correction information Km.

Next, a second embodiment of the present invention will be described.

In the same manner as the high-frequency treatment device 1 according to the first embodiment, a high-frequency treatment device 2 according to the second embodiment is for performing nerve blocking by partially heating a peripheral nerve with a high-frequency current. The high-frequency treatment device 2 basically has the same configuration as the high-frequency treatment device 1 except that the high-frequency treatment device 2 includes only three of the electrode units 31, 32, and 33 and a different internal configuration. Therefore, the same reference numerals will be given to the same portions as in the first embodiment, and the description thereof will be omitted, and only the portions different from the first embodiment will be explained, hereinafter.

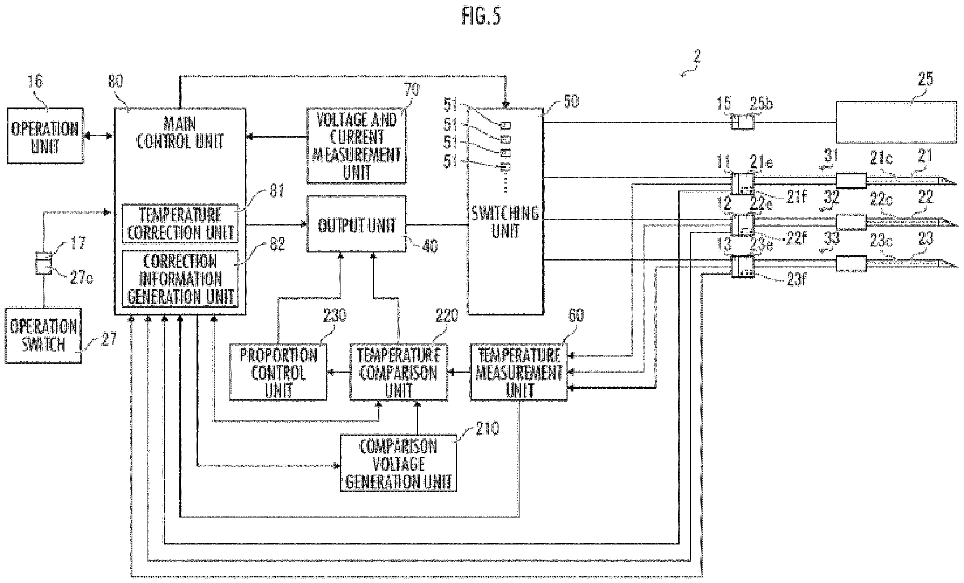

FIG. 5 is a block diagram illustrating the internal configuration of the high-frequency treatment device 2. As illustrated in FIG. 5, the high-frequency treatment device 2 has, as the internal configuration, an output unit 40, the switching unit 50, a temperature measurement unit 60, a comparison voltage generation unit 210, a temperature comparison unit 220, a proportion control unit 230, a voltage and current measurement unit 70, and a main control unit 80.

With a configuration in the same manner as the first output unit 41 and the second output unit 42, based on power supplied from the commercial alternating current power supply, the output unit 40 generates and outputs high-frequency power with a preset frequency (for example, 470 to 490 kHz) and a voltage based on an output control signal from the main control unit 80 (for example, 18 to 22 Vrms). The output unit 40 is connected to the main body-side connectors 11 to 15 via the switching unit 50, that is, is connected to be capable of outputting the high-frequency power to the electrodes 21 to 23 and the counter electrode plate 25.

In the same manner as the first embodiment, the switching unit 50 is configured with a circuit including the plurality of switches 51 including a semiconductor switch such as a MOSFET or a reed relay, and is provided between the output unit 40, and the electrodes 21 to 23 and the counter electrode plate 25. In the present embodiment, the switching unit 50 operates under control of the main control unit 80, and switches the connection between the output unit 40, and the main body-side connectors 11 to 15.

The temperature measurement unit 60 has a configuration in the same manner as the first temperature measurement unit 61 in the first embodiment, and generates, for example, a temperature measurement signal of 40 mV/°C and transmits the temperature measurement signal to the main control unit 80, and generates, for example, a temperature measurement signal of 20 mV/° C and transmits the temperature measurement signal to the temperature comparison unit 220, based on a signal received from the thermocouples 21c to 23c.

The comparison voltage generation unit 210 is configured with a known circuit or the like, and generates a control reference voltage signal to be used for output control of the output unit 40 and a determination reference voltage signal to be used for determining an abnormality in a measurement temperature. The comparison voltage generation unit 210 is controlled by the main control unit 80 to generate the control reference voltage signal (voltage indicating a control target value) based on the control target value received from the main control unit 80, and transmits the control reference voltage signal to the temperature comparison unit 220. The comparison voltage generation unit 210 also generates the determination reference voltage signal (for example, a voltage indicating a temperature setting value of +7°C) based on the temperature setting value received from the main control unit 80, and transmits the determination reference voltage signal to the temperature comparison unit 220.

The temperature comparison unit 220 is configured with a known circuit or the like, and transmits a difference voltage signal indicating a voltage difference between the control reference voltage signal received from the comparison voltage generation unit 210 and the correction temperature signal received from the main control unit 80 to the proportion control unit 230. The temperature comparison unit 220 also compares a voltage of the determination reference voltage signal received from the comparison voltage generation unit 210 with a voltage of the temperature measurement signal received from the temperature measurement unit 60, and transmits an output stop signal to the output unit 40 and the main control unit 80 in a case where the voltage of the temperature measurement signal is higher. The output unit 40 that receives the output stop signal stops the output of the high-frequency power, and the main control unit 80 that receives the output stop signal performs a temperature abnormality process such as an alarm display.

The proportion control unit 230 is configured with a known PWM circuit including a sawtooth wave transmitter and a comparator, and compares the difference voltage signal received from the temperature comparison unit 220 with a sawtooth wave signal to generate a pulse signal, and transmits the pulse signal to the output unit 40. The output unit 40 outputs high-frequency power having a voltage based on a temperature control signal obtained by synthesizing the pulse signal received from the proportion control unit 230 and the output control signal received from the main control unit 80.

The voltage and current measurement unit 70 is configured with a known circuit or the like, and measures a voltage and a current of the high-frequency power output by the output unit 40. The voltage and current measurement unit 70 generates a voltage measurement signal and a current measurement signal based on a high-frequency voltage and a high-frequency current generated by the output unit 40, and transmits the voltage measurement signal and the current measurement signal to the main control unit 80.

The main control unit 80 includes a known configuration of a CPU, a ROM, a RAM, and the like, and controls each unit of the high-frequency treatment device 2 to execute a high-frequency treatment, in the same manner as the first embodiment. The main control unit 80 calculates a temperature measurement value based on the temperature measurement signal received from the temperature measurement unit 60. The main control unit 80 also generates an output control signal based on the calculated temperature measurement value and transmits the output control signal to the output unit 40 to control the output of the high-frequency power together with the proportion control unit 230.

In the present embodiment, the main control unit 80 includes a temperature correction unit 81 and a correction information generation unit 92 having the same functional configuration as the temperature correction unit 91 and the correction information generation unit 92 of the first embodiment. Therefore, in the calculation of the temperature measurement value, the temperature correction unit 81 corrects the temperature measurement signal with the first correction information Ke and the second correction information Km. In addition, the main control unit 80 transmits the correction temperature signal generated by the temperature correction unit 81 to the temperature comparison unit 220. The correction information generation unit 82 generates the second correction information Km by the method illustrated in the first embodiment, and stores the second correction information Km in a storage device such as a flash memory included in the main control unit 80.

In addition, the main control unit 80 calculates a voltage measurement value, a current measurement value, a power measurement value, and an impedance measurement value based on the received voltage measurement signal and current measurement signal. Further, in the present embodiment, the main control unit 80 controls the switching unit 50. Specifically, based on an input operation accepted by the operation unit 16, the main control unit 80 controls ON and OFF of the plurality of switches 51 included in the switching unit 50, and switches a connection state of the output unit 40, and the main body-side connectors 11 to 15.

Next, an operation of the high-frequency treatment device 2 will be described.

The high-frequency treatment device 2 is capable of three types of output formats of a high-frequency current to a treatment target: monopolar, bipolar, and tripolar. In the present embodiment, the monopolar manner has an output format in which a high-frequency current flows through an electrode set in which any one of the electrodes 21 to 23 and the counter electrode plate 25 are combined, and the bipolar manner has an output format in which a high-frequency current flows by using the electrodes 21 and 22 as an electrode set.

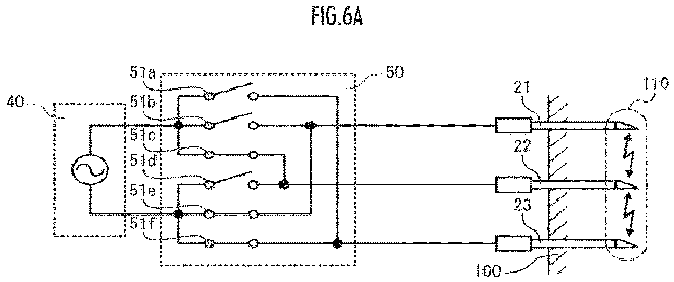

Hereinafter, the operation of the high-frequency treatment device 2 will be described by using a case where an output format is tripolar, as an example. FIGS. 6A to 6C are schematic views illustrating an operation state in a tripolar manner. In order to facilitate understanding, FIGS. 6A to 6C describe only the switches 51a to 51f necessary for description.

When local anesthesia for reducing pain due to overheating during a treatment and arrangement of the electrodes 21 to 23 are appropriately performed and the operation unit 16 accepts a tripolar manner selection operation by a user such as a doctor, the main control unit 80 controls the switching unit 50 to set a connection state between the output unit 40 and the electrodes 21 to 23 to be a connection state illustrated in FIG. 6A. Specifically, the main control unit 80 turns on the switch 51c, the switch 51e, and the switch 51f, and turns off the switch 51a, the switch 51b, and the switch 51d. Accordingly, with high-frequency power output by the output unit 40, a state is obtained in which a high-frequency current flows between the electrode 22, and the electrodes 21 and 23 in an electrode set configured with the three electrodes 21 to 23.

After that, when the operation unit 16 accepts a start operation of a treatment after the operation unit 16 receives an operation of selecting the high-frequency thermal coagulation method or the pulse high-frequency method and an input operation of a temperature setting value (for example, 80°C) and a treatment time (for example, 3 minutes) by the user (in a case where the pulse high-frequency method is selected, the main control unit 80 sets the temperature setting value to 42°C, so that only the treatment time is input), the main control unit 80 controls the output unit 40 to start an output of high-frequency power.

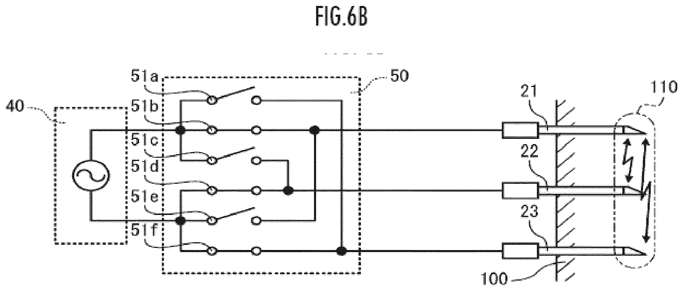

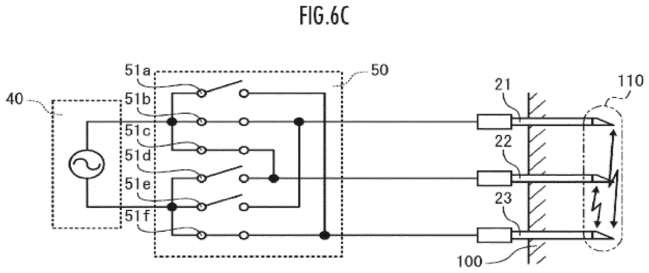

Further, the main control unit 80 controls the switching unit 50 to switch the connection state of the output unit 40 with the electrodes 21 to 23 between three states illustrated in FIGS. 6A to 6C in a specific cycle (0.1 seconds in the present embodiment). Specifically, the state illustrated in FIG. 6B is a state in which the switch 51b, the switch 51d, and the switch 51f are turned on and the switch 51a, the switch 51c, and the switch 51e are turned off, so a high-frequency current flows between the electrode 21, and the electrode 22 and the electrode 23. Further, the state illustrated in FIG. 6C is a state in which the switch 51b, the switch 51c, and the switch 51f are turned on and the switch 51a, the switch 51d, and the switch 51e are turned off, so a high-frequency current flows between the electrode 23, and the electrode 21 and the electrode 22.

That is, during the output of the high-frequency power, the main control unit 80 controls the switching unit 50 to switch any one of the electrodes 21 to 23 in order at a specific cycle so that a high-frequency current flows between the remaining two electrodes. Accordingly, the region 110 between the electrodes 21 and 23 in the periphery of the electrodes 21 and 23 is heated.

After the calculated temperature measurement value is increased up to a temperature substantially equal to the control target value, the main control unit 80 controls the output of the output unit 40 to maintain a state in which the temperature measurement value is substantially equal to the control target value, together with the proportion control unit 230. Further, the main control unit 80 changes the control target value according to the presence or absence of detection of an operation by the operation switch 27, and switches between the normal output control and the output reduction control.

Also in the present embodiment, by correcting the temperature measurement signal by the first correction information Ke and the second correction information Km, it is possible to cancel an error caused by individual differences of the components related to the temperature measurement in the electrode unit 31 and the main body 10. Accordingly, it is possible to measure an ambient temperature of the electrodes 21 to 24 with high accuracy and control the output of the high-frequency power. Therefore, it is possible to accurately maintain a preset treatment temperature for heating the nerve tissue and appropriately perform the treatment.

In the present embodiment, based on the temperature measurement value of the thermocouple 22c incorporated in the electrode 22 in the state illustrated in FIG. 6A, the temperature measurement value of the thermocouple 21c incorporated in the electrode 21 in the state illustrated in FIG. 6B, and the temperature measurement value of the thermocouple 23c incorporated in the electrode 23 in the state illustrated in FIG. 6C, the output control is performed by the main control unit 80 and the proportion control unit 230. That is, in the present embodiment, accuracy and stability of the output control are improved by referring to an ambient temperature of an electrode having the highest current density among the electrodes 21 to 23.

Although the embodiments of the present invention are described above, the high-frequency treatment device and the high-frequency treatment method of the present invention are not limited to the embodiments described above, and are variously modified without departing from the gist of the present invention.

For example, the shape and arrangement of each unit of the high-frequency treatment devices 1 and 2 are not limited to the shapes and arrangements illustrated in the embodiments described above, and it is possible to adopt various known shapes and arrangements. In addition, the high-frequency treatment devices 1 and 2 are not limited to the apparatuses that perform nerve blocking, and may be used for other purposes such as ablation of a tumor, for example.

In addition, the number of each configuration included in the high-frequency treatment devices 1 and 2 is not limited to the numbers illustrated in the embodiments described above, and it is possible to adopt any number. For example, in the high-frequency treatment device 1, the second output unit 42 may be omitted and high-frequency power may be output from the first output unit 41 to the electrodes 21 to 24 and the counter electrode plate 25, or the number of electrodes may be increased to 6 and a third output unit and a third temperature measurement unit may be provided at the same time. Further, in the high-frequency treatment device 2, the two output units 40 and the two temperature measurement units 60 may be provided, or the sub-control unit 90 that controls the switching unit 50, the temperature measurement unit 60, and the like may be provided.

Further, the electrodes 21 to 24 are not limited to electrodes inserted into the treatment target, and may be, for example, disposed on a skin surface of a human body to heat a relatively shallow region under the skin. In addition, the number of electrodes constituting the electrode set is not particularly limited as long as the number of electrodes is two or more. Further, the operation switch 27 and the process related to the operation switch 27 may be omitted.

In addition, the actions and effects illustrated in the embodiments described above are merely a list of the most appropriate actions and effects resulting from the present invention, and the actions and effects according to the present invention are not limited thereto.

### Description of Reference Numerals

1, 2 high-frequency treatment device
10 main body
21 to 24 electrode
21c to 24c thermocouple
21f to 24f storage device
31 to 34 electrode unit
40 output unit
41 first output unit
42 second output unit
60 temperature measurement unit
61 first temperature measurement unit
62 second temperature measurement unit
80 main control unit
81 temperature correction unit
92 correction information generation unit
90 sub-control unit
91 temperature correction unit
92 correction information generation unit
100 treatment target

## Claims

1. A high-frequency treatment device comprising:
an output unit configured to output high-frequency power;
a plurality of electrodes connected to the output unit and disposed at a treatment target;
a temperature sensor provided at the electrode or disposed in a vicinity of the electrode;
a temperature measurement unit configured to generate a temperature measurement signal based on a signal received from the temperature sensor;
a temperature correction unit configured to correct the temperature measurement signal based on correction information on an individual characteristic of a component related to the temperature measurement to generate a correction temperature signal or calculate a temperature measurement value; and
a control unit configured to control the output of the output unit based on the correction temperature signal or the temperature measurement value.

2. The high-frequency treatment device according to claim 1, further comprising:
a main body configured to include the output unit, the temperature measurement unit, the temperature correction unit, and the control unit; and
an electrode unit configured to include the electrode and the temperature sensor and be detachably connected to the main body,
wherein the correction information includes first correction information on an individual characteristic of a component related to the temperature measurement in the electrode unit.

3. The high-frequency treatment device according to claim 2,
wherein the correction information includes second correction information on an individual characteristic of a component related to the temperature measurement in the main body.

4. The high-frequency treatment device according to claim 2 or 3,
wherein the electrode unit includes a storage device configured to store the first correction information.

5. The high-frequency treatment device according to claim 1, further comprising:
a main body configured to include the output unit, the temperature measurement unit, the temperature correction unit, and the control unit; and
an electrode unit configured to include the electrode and the temperature sensor and be detachably connected to the main body,
wherein the correction information includes second correction information on an individual characteristic of a component related to the temperature measurement in the main body.

6. The high-frequency treatment device according to claim 5,
wherein the correction information includes first correction information on an individual characteristic of a component related to the temperature measurement in the electrode unit.

7. The high-frequency treatment device according to claim 5 or 6,
wherein the main body includes a correction information generation unit configured to generate the second correction information.

8. A high-frequency treatment method of outputting high-frequency power to a plurality of electrodes disposed at a treatment target to perform a treatment, the method comprising:
a temperature measurement process of generating a temperature measurement signal based on a signal received from a temperature sensor provided at the electrode or disposed in a vicinity of the electrode;
a temperature correction process of correcting the temperature measurement signal based on correction information on an individual characteristic of a component related to the temperature measurement to generate a correction temperature signal or calculate a temperature measurement value; and
an output control process of controlling the output of the high-frequency power based on the correction temperature signal or the temperature measurement value.
